# EUROPEAN PATENT SPECIFICATION

(11) **EP 3 924 712 B1**
(45) Date of publication and mention of the grant of the patent: **16.10.2024**
(21) Application number: 20755649.9
(22) Date of filing: 13.02.2020
(51) Int. Cl.: G01N 1/31, G01N 1/40, C12M 1/24

(54) **MEDICAL APPARATUS FOR COLLECTION AND PREPARATION OF BIOLOGICAL SAMPLES**
MEDIZINISCHES GERÄT ZUR ENTNAHME UND VORBEREITUNG VON BIOLOGISCHEN PROBEN
APPAREIL MÉDICAL DE PRÉLÈVEMENT ET DE PRÉPARATION D'ÉCHANTILLONS BIOLOGIQUES

(30) Priority: 13.02.2019 US 201962804875 P; 27.01.2020 US 202062966264 P
(43) Date of publication of application: 22.12.2021
(73) Proprietor: The Trustees of Columbia University in the City of New York, New York, NY 10027 (US)
(72) Inventor: SAQI, Anjali, NY 10069 (US); YEAGER, Keith, NY 10027 (US)
(74) Representative: Berggren Oy
(86) International application number: PCT/US2020/018121
(87) International publication number: WO 2020/168085

(56) References cited:
- WO-A1-2013/059526
- WO-A1-2016/028997
- WO-A1-2019/001228
- CN-A- 1 321 106
- CN-U- 201 825 948
- US-A1- 2012 271 254
- US-A1- 2014 227 732
- US-A1- 2017 036 203
- US-A1- 2017 087 228
- US-B2- 7 488 297

## Description

### FIELD

The disclosed subject matter relates to a system, apparatus and method for preparing cells and small tissue samples for diagnostic/research tests and procedures.

### BACKGROUND

Medicine is becoming less invasive and more personalized. For example, a patient presenting with a mass in the lung or pancreas is not necessarily scheduled for surgery to characterize the lesion as neoplastic or not. Instead, a minute sample of cells from the lesion is obtained through a minimally invasive procedure (MIP) called a core biopsy and/or fine needle aspiration (FNA). Both of these MIPs involve obtaining a sample with a small caliber needle after it is localized to the site of interest with the aid of imaging, such as a CT scan and/or ultrasound. When performing MIP, either no incision is made, or the biopsy site is inconspicuous, similar to a puncture wound following a blood draw, which allows for outpatient procedures and prevents need for hospitalization. By examining the sample obtained with a MIP under a microscope, pathologists render diagnoses of benignity or malignancy. At one time, there were limited treatment options and diagnoses of malignancy made simply by morphology assessed under a microscope would suffice and treatment would ensue. Nowadays, ancillary tests afford greater information than morphology alone about the tumor and therapeutic options that are likely to be more effective and personalized. Though MIPs and personalized treatment options provide better patient care, there is inconsistency among laboratories when imparting greater levels of information on such small tissue samples. Managing these small samples can be challenging, places a greater burden on laboratories/pathologists and has consequences for patients.

Ancillary tests to answer the pertinent questions are frequently conducted on small samples, including biopsies and cell blocks (cell pellets formed from cytology samples such as FNAs sample often with the aid of centrifugation). Currently, there is no accepted laboratory standard on the preparation of cell blocks, though labs frequently employ one of several "homebrew" methods. When samples are large, cell blocks are easier to form, but with smaller samples, the "homebrew" methods may fail or result in a suboptimal cell block. Thus, there is a growing need to develop a standardized apparatus and method for preparing cell blocks in a low cost and efficient manner to provide answers to clinicians that impact therapeutic decisions.

WO 2019/001228 A1 describes a fixed tube of a nucleic acid extraction component and a nucleic acid extraction component. The nucleic acid extraction component comprises a cylindrical membrane tube, which is fitted over the fixed tube; the fixed tube comprises a tube body, a tube opening, and a bottom; the tube body extends along a first direction; the end of the tube opening distal from the tube body is provided with a protrusion which protrudes along a second direction, the first direction being vertical to the second direction; the bottom and the tube opening are respectively connected to two opposite sides of the tube body; the bottom is provided with a shoulder; and the shoulder of the bottom is connected to the cylindrical membrane tube.

WO 2013/059526 A1 describes a medical apparatus and method of preparing one or more cell blocks. The medical apparatus comprises at least one elongate tubular body having a proximal end and a distal end and a filter membrane disposed between the proximal end and a distal end of the elongate tubular body. The filter membrane, which can include alignment features and structural features to engage the tubular body, and/or cover, is sectionable. Additionally, in some embodiments a second elongate tubular body is provided which telescopingly receives at least a portion of the first elongate tubular body. In other embodiments, a support member is provided for engaging the filter membrane and positioning the filter membrane at a midpoint of the elongate tubular body during centrifuging.

### SUMMARY

The purpose and advantages of the disclosed subject matter will be set forth in and apparent from the description that follows, as well as will be learned by practice of the disclosed subject matter.

The invention relates to a medical apparatus as defined in claim 1. Further embodiments of the invention are defined in the dependent claims.

The invention relates to a medical apparatus including an elongated body and defining a loading chamber between a proximal end and a distal end thereof for storing a liquid therein, the elongated body defining an outlet at the distal end thereof; a cell block filter assembly including a cell block body and defining a well portion matingly engageable with the distal end of the elongated body for receiving fluid from the outlet of the elongated body and defining a well opening on a bottom thereof; a filter membrane disposed across the well opening on the bottom of the well portion; and a cover member positionable over the well portion wherein the entire cell block filter assembly is sliceable into slices having a thickness suitable for mounting on a laboratory slide.

The medical apparatus further includes a base member for securing the cell block filter assembly at the distal end of the elongate portion, and wherein the outer surface of the distal end of the elongate body is configured to engage with the base member, wherein the base member prevents the passage of fluid out of the filter assembly. The base member can be secured to the elongate body with threaded engagement or a ratchet engagement.

In some embodiments, the cell block body is fabricated substantially from LLDPE. The cover can fabricated substantially from LLDPE. In some embodiments, the cover defines a cover opening, the cover further comprising a filter membrane disposed across the cover opening.

In some embodiments, the filter membrane is fabricated substantially from ePTFE,

The distal end portion of the elongate body defines a smaller aperture than the proximal portion to define a generally conical portion in some embodiments. The outlet can be offset from the longitudinal axis of the elongated body.

In some embodiments, the medical apparatus includes a lid disposed on the proximal end of the elongated body, the lid configured to engage the proximal end of the elongate body to form an airtight seal between the lid and the elongated body.

In some embodiments, the lid is threadedly engaged with the elongated body by threads formed on the outer surface of the lid and by threads formed on the inner surface of the proximal end of the elongated body.

In some embodiments, the outlet is sized to prevent fluid from flowing from the loading chamber when the elongated body is positioned such that the outlet and the fluid are disposed at a bottom portion of the elongated body.

The medical apparatus can include a cell block filter assembly for preparing a cell block formed at least in part from the condensed biological sample. The cell block filter assembly is formed from a material that is sliceable by for example, a blade such as a microtome blade, into slices having a thickness suitable for mounting on a slide for microscopy or other laboratory analyses. A cell block filter assembly is provided including a body defining a well for receiving a biological sample, the body fabricated substantially from low-density polyethylene (LLDPE); and a filter membrane disposed across an aperture in the bottom portion of the well and defining pores therethrough, the filter membrane being fabricated substantially from expandable polytetrafluoroethylene (ePTFE) wherein the entire cell block filter assembly is sliceable into slices having a thickness suitable for mounting on a laboratory slide.

Both the filter membrane and frame components of the cell block filter assembly are designed to both hold a prepared cell block and be sliceable by a blade (such as a microtome blade) to form slices containing both the cell block and the cell block assembly. In this regard, the cross section of the slice contains cell block and surrounded by the sliced cell block filter assembly because the assembly is designed so that the cell block need not be removed from the cell block assembly for laboratory processing. This advantageously concentrates and confines the sample. Also, this reduces waste and loss of cells, which can occur if the cells were transferred into a paraffin mold from the cell block filter assembly with a forceps-the tool typically used in the laboratory. The sectionable slices are suitable for mounting on a slide for microscopy or for other laboratory analyses. In some embodiments, the slicing of the cell block filter assembly containing a cell block results in a ribbon comprising a plurality of interconnected slices (of cell block and filter assembly) wherein each of the plurality of slices are separatable from each other for mounting on a microscopic slide or other laboratory analyses.

In another embodiment, a medical apparatus is provided including an elongated body and defining a loading chamber between a proximal end and a distal end thereof for storing a liquid therein, the elongated body defining an outlet at the distal end thereof; a cell block filter assembly including a cell block body and defining a well portion matingly engageable with the distal end of the elongated body for receiving fluid from the outlet of the elongated body and defining a well opening on a bottom thereof; a filter membrane disposed across the well opening on the bottom of the well portion; a cover member positionable over the well portion, and a hinge portion connecting the cover and the cell block body, wherein the entire cell block filter assembly is sliceable into slices having a thickness suitable for mounting on a laboratory slide.

In the some embodiments, the hinge portion is a "living hinge."

Additionally, the apparatus or select components thereof can be disposable, or designed for repeated use and cleansing.

It is to be understood that both the foregoing general description and the following detailed description are exemplary and are intended to provide further explanation of the disclosed subject matter claimed.

The accompanying drawings, which are incorporated in and constitute part of this specification, are included to illustrate and provide a further understanding of the method and system of the disclosed subject matter. Together with the description, the drawings serve to explain the principles of the disclosed subject matter.

### BRIEF DESCRIPTION OF THE DRAWINGS

A detailed description of various aspects, features, and embodiments of the subject matter described herein is provided with reference to the accompanying drawings, which are briefly described below. The drawings are illustrative and are not necessarily drawn to scale, with some components and features being exaggerated for clarity. The drawings illustrate various aspects and features of the present subject matter and may illustrate one or more embodiment(s) or example(s) of the present subject matter in whole or in part.
FIG. 1 is a schematic representation of a side view of an exemplary embodiment of the disclosed subject matter with components separated.
FIG. 2 is a cross-sectional view of the embodiment of FIG. 1 with components separated.
FIGS. 3-4 are perspective views of the embodiment of FIG. 1 with components separated.
FIG. 5 is an enlarged cross-sectional view of the embodiment of FIG. 1 with components separated.
FIG. 6 is a side view of the embodiment of FIG. 1 with the components assembled.
FIG. 7 is a perspective view of the embodiment of FIG. 1 with components assembled.
FIG. 8 is a cross-sectional view of the embodiment of FIG. 1 with the components assembled.
FIG. 9 is an enlarged cross-sectional view of the embodiment of FIG. 1 with components assembled.
FIG. 10 is a perspective view, from above, of a component of the embodiment of FIG. 1.
FIG. 11 is a perspective view in partial cross-section, from above, of the component of FIG. 10, in accordance with an exemplary embodiment of the disclosed subject matter.
FIG. 12 is a perspective view, from below, of the component of FIG. 10, in accordance with an exemplary embodiment of the disclosed subject matter.
FIG. 13 is a perspective view, from above, of the component of FIG. 10 with an additional cover component, in accordance with an exemplary embodiment of the disclosed subject matter.
FIG. 14 is a perspective view in partial cross-section, of the components of FIG. 13, in accordance with an exemplary embodiment of the disclosed subject matter.
FIG. 15 is a side view of the embodiment of FIG. 1, with selected components separated, in accordance with an exemplary embodiment of the disclosed subject matter.
FIG. 16 is a cross-sectional view of the embodiment of FIG. 1, with selected components separated, in accordance with an exemplary embodiment of the disclosed subject matter.
FIG. 17 is a perspective view, from above, of a component of the embodiment of FIG. 1, in accordance with another exemplary embodiment of the disclosed subject matter.
FIG. 18 is a perspective view in partial cross-section, of the component of FIG. 17, in accordance with an exemplary embodiment of the disclosed subject matter.
FIG. 19 is a perspective view, from below, of the component of FIG. 17, in accordance with an exemplary embodiment of the disclosed subject matter.
FIG. 20 is a perspective view of another exemplary embodiment of the disclosed subject matter.
FIG. 21 is a cross-sectional view of the embodiment of FIG. 20.
FIG. 22 is a perspective view from below of the embodiment of FIG. 20 with certain components removed.
FIG. 23 is an enlarged cross-sectional view of the embodiment of FIG. 20.
FIG. 24 is a perspective view from the side of a component of the embodiment of FIG. 20.
FIG. 25 is a perspective view from the top of a component of the embodiment of FIG. 20.
FIG. 26 is a top view of a component of the embodiment of FIG. 20.
FIG. 27 is a perspective view in partial cross-section of a component of the embodiment of FIG. 20 taken from line 27-27 of FIG. 25.

### DETAILED DESCRIPTION

Reference will now be made in detail to select embodiments of the disclosed subject matter, examples of which are illustrated in the accompanying drawings. The method and corresponding steps of the disclosed subject matter will be described in conjunction with the detailed description of the system.

Unless defined otherwise, all technical and scientific terms used herein have the same meaning as commonly understood by one of ordinary skill in the art to which this disclosed subject matter belongs. Although any methods and materials similar or equivalent to those described herein can also be used in the practice or testing of the present disclosed subject matter, this disclosure may specifically mention certain exemplary methods and materials.

As used herein and in the appended claims, the singular forms "a," "an," and "the" include plural referents unless the context clearly dictates otherwise.

In accordance with the various embodiments of the disclosed subject matter, as summarized above and as described in further detail below, there is provided an apparatus for collecting and separating a liquid component from a cellular, or solid particle component, of a biological sample. While an exemplary embodiment disclosed herein includes fine needle aspiration, the apparatus and method of the disclosed subject matter is not limited to this exemplary embodiment and will be understood by an artisan of ordinary skill to be operable for collection and separation of any bodily fluids or specimens. In an exemplary embodiment, a disposable cell block system and a method for using the system, e.g., for tumor diagnosis, benign diagnosis, and other ancillary tests including research and development analyses, is provided. As used herein, the term "cell block" refers to a concentration of cells or solid particles from a biological sample, which is embedded in a medium, such as but not limited to paraffin wax. Thin slices from the medium with embedded cells are sliceable together with the cell block assembly in sizes suitable for mounting on a glass slide for analysis on a microscope or for other analyses. For example, visualization of the cells and the extracellular environment can provide information to determine whether the tissue collected is benign or malignant. Alternatively, the slices provide cellular material (DNA, RNA, proteins) for microcellular analysis. Although particular embodiments disclosed herein may focus on collection of the tissue or solid particle component in a biological sample for further diagnostics/testing, it will be understood by one of ordinary skill in the art that the disclosed system, apparatus and method is equally applicable for applications in which the fluid component of the biological sample is to be the subject of further diagnostics/testing/research.

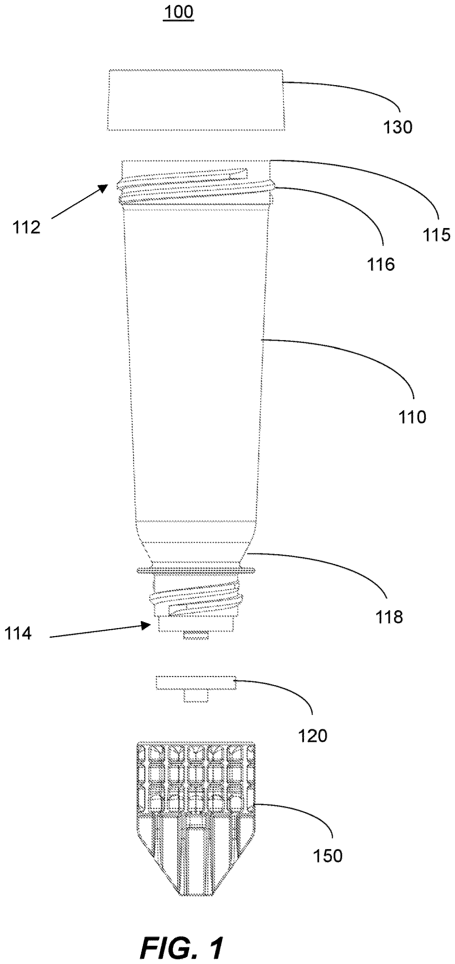

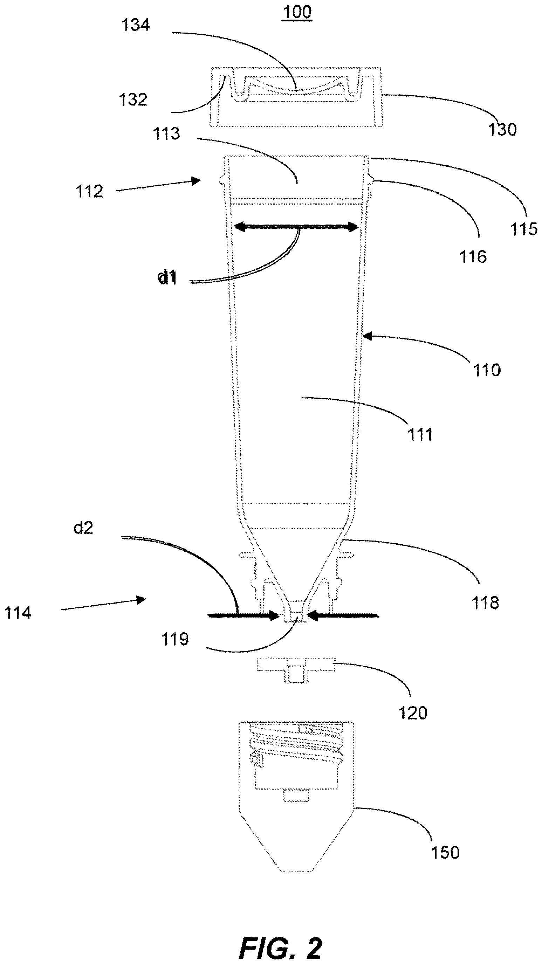

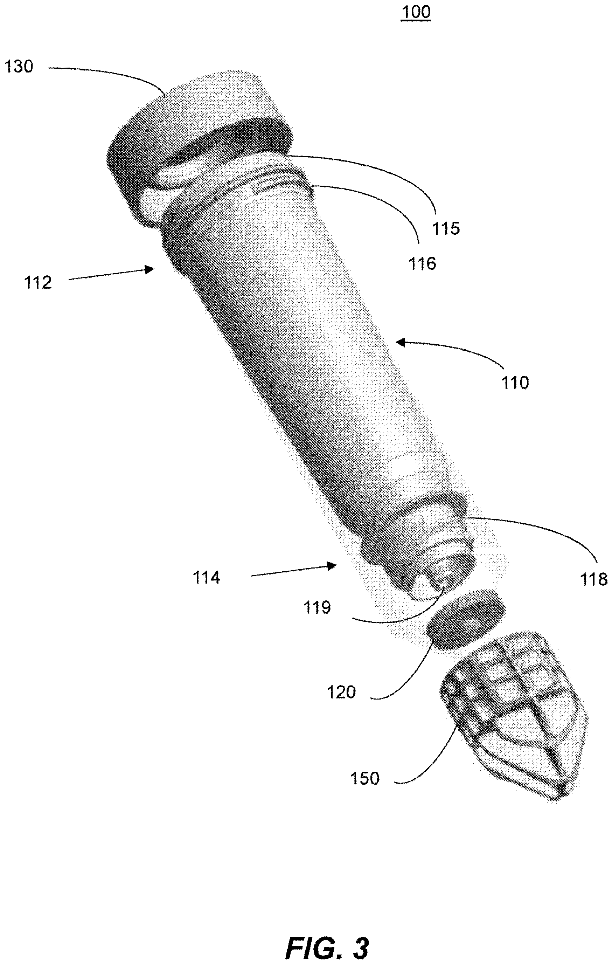

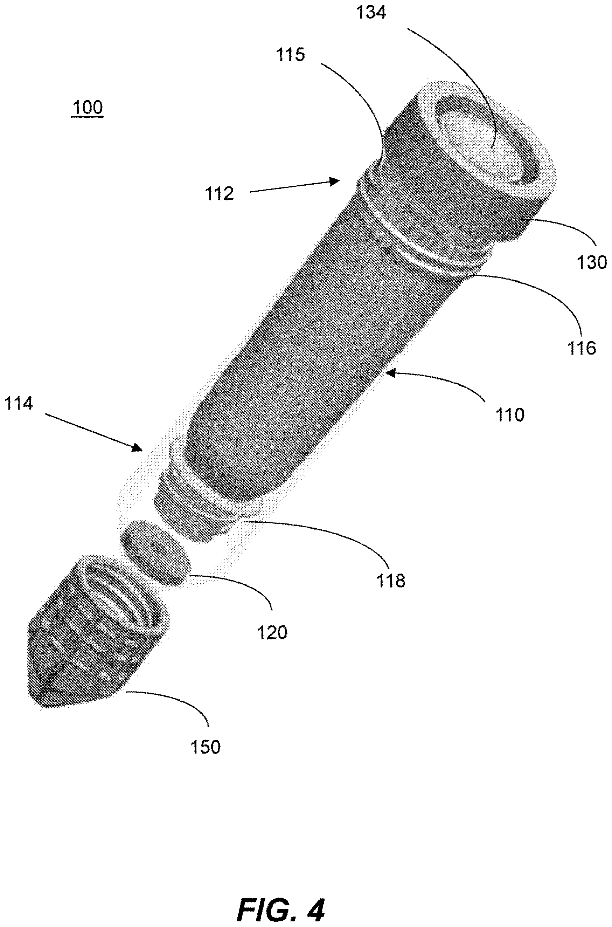

In one exemplary embodiment, the cell block system 100 is shown schematically in FIGS. 1-4. Cell block system 100 includes an elongate tubular body 110, a cell block filter assembly 120, and a base member 150. The elongate tubular body 110 defines an interior loading chamber 111 (See FIG. 2), and has a proximal end 112 and a distal end 114. In some embodiments, the elongate tubular body 110 has a first diameter (d1) at the proximal end and a second diameter (d2) at the distal end, wherein the second diameter is smaller than the first diameter, as will be discussed in greater detail herein. A section 118 disposed between the proximal end 112 and the distal end 114 of the elongate tubular body 110, has a decreasing diameter along a length thereof to define a generally conical configuration, and further includes structure for receiving the cell block filter assembly 120 as will be described below.

Various suitable volumes are available for elongate tubular body 110. For purpose of illustration and not limitation, suitable volumes include between about 15 ml to about 50 ml, or any other size that fits into a centrifuge, standard or otherwise. However, it will be understood by one of ordinary skill in the art that alternative sizes are within the scope of the disclosed subject matter. The elongate tubular body is sized to fit within a conventional centrifuge. In this manner, the cell block apparatus can receive the biological sample, for example, from a needle housing the biological sample obtained by fine needle aspiration techniques, and is disposed in the centrifuge for stratification/separation of the cells in the biological sample from any liquid to isolate and consolidate the cells into a concentrated pellet by centrifugation. Using the same unit for receiving the biological sample and stratifying/separating the biological sample into component parts reduces the loss of sample size and reduces risk of contamination due to exchange between multiple components. In contrast, the most common and typical practice among technicians is to use a single pipette for multiple components, thus allowing contamination between components, the instrument most commonly and typically utilized. In some embodiments, the elongate tubular body is suitable for relative centrifugal forces of between about 1,200 to about 16,000 RCF. For example, 12,000 RCF, 1,200 RCF, 16,000 RCF, 2,000 RCF, 9,400 RCF, 7,500 RCF. For further illustration in one embodiment, the elongate tubular member has a volume of 15 ml, and is suitable for centrifugation at 1,200 RCF or 12,000 RCF. In other embodiments, for example, the elongate tubular member has a volume of 50 ml and is suitable for centrifugation at 16,000 RCF or 2,000 RCF or 9,400 RCF. The elongate tubular body of the device can be formed of various materials and in particular various polymers, for example, polypropylene and/or polystyrene. Further, the materials used for the elongate tubular body, filter assembly, or compressive cover or cap (the terms are used interchangeably herein), which is described below, can be biodegradable material.

With continued reference to FIGS. 1-4, the elongate tubular body 110 defines an opening 113 having a rim 115 at the proximal end 112 of the body. The opening 113 is closed by a lid 130, which provides a substantially airtight seal with the rim 115. The lid 130 can be configured with a screw thread or threads on an inner surface (not shown) to engage a thread or threads 116 disposed on the outside surface of a proximal section 112 of the elongate tubular body 110. Alternatively, the cover may comprise screw thread or threads disposed on an outside surface to engage thread or threads on the inner surface of the elongate tubular body at the proximal end so that the cover engages the elongate tubular body in the interior space. However, other suitable methods and features can be used to engage the lid 130 and elongate tubular body 110, such as a snap fit, ratchet fit, interference fit, vacuum seal or other methods of engagement, as would be appreciated by one of ordinary skill in the art.

In some embodiments, the elongate tubular body 110 is preloaded with a fixative in the loading chamber 111. A "fixative" as used herein refers to a compound, such as formalin, ethanol, methanol, RPMI, saline for preservation and/or transportation of the sample.

When the lid 130 is engaged with the rim 115, a substantially airtight seal is provided. As will be discussed further below, the distal outlet 119 is sized such that any liquid, such as the fixative discussed herein, disposed within the loading chamber 111 will be retained therein and prevented from flowing out of the distal outlet 119 when the elongated body 110 is positioned such that the aperture 119 and the fluid are disposed at a bottom portion of the elongated body. This feature is particularly useful when it is desirable to reuse the liquid for further experiments. In an embodiment, the lid 130 includes a seal 132 on the inner periphery that engages with the rim 115 to provide the airtight seal. The distal outlet 119 has a diameter in the range of about 12.5 mm at the maximum to about 1 mm at the minimum. In some embodiments, the distal outlet 119 has a diameter of about 8-9 mm. Distal outlet 119 has a circular shape, although oval or rectangular openings are contemplated.

In an embodiment, lid 130 further comprises a central portion 134 formed from a self-sealing or resealable material that overlays the opening 113. In an alternative embodiment, the cover can be a stopper formed from a self-sealing or resealable material that can be inserted into the opening 113 at the proximal end 112. The central portion 134 is puncturable by a needle allowing transfer of the biological sample from the needle to the interior of the elongate tubular body. After deposit of the biological sample and removal of the needle from the lid 130, the material self-seals the puncture created by the needle entry and maintains an airtight seal.

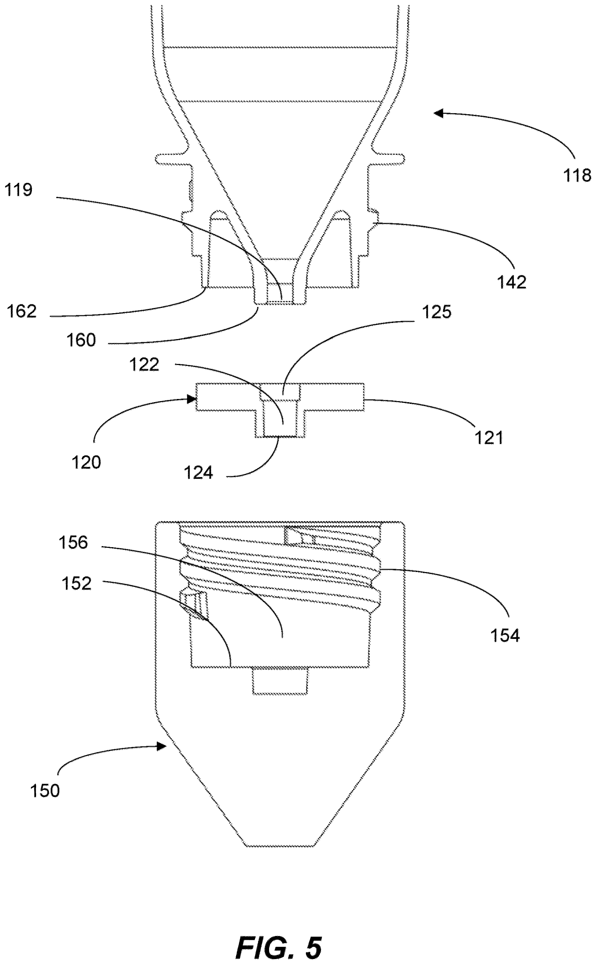

As illustrated in FIG. 5, at the distal section 118 of the elongate tubular body 110 is configured to permit the engagement and secure mounting of the cell block filter assembly 120 thereto. The filter assembly 120 defines a border 121 circumscribing an open center well 122 in which a filter membrane 124 is disposed. The well 122 is configured with an opening 125 sized to cooperate with an inner flange 160 circumscribing the distal outlet 119 on the distal end 114 of the elongate tubular body 110. The filter assembly 120 is matingly engageable with the elgonated tubular body 110, e.g., to allow fluid to flow from the loading chamber 111 into the well 122. Additionally or alternatively, the periphery of the border 121 of the filter assembly 120 is configured to receive and releasably engage the distal portion 118 of the elongate tubular body 110, e.g., at outer flange 162. In an embodiment, cells may be collected in the center well 122, such as after filtration, to provide a substantially cylindrical cell block.

The base member 150 is configured to support the cell block filter assembly 120 at surface 152, and to be attached to the distal section 118 of tubular body 110 to secure the assembly 120 thereto. In some embodiments, the outer surface of the distal section 118 can be configured with a thread or a plurality of threads 142 to engage with a thread or threads 154 on interior portion of the base member 150 to secure the base member 150 to the distal section 118 of the elongate tubular body 110, thereby stabilizing the cell block filter assembly 120 therein during centrifuging or other similar processes. It is contemplated that other techniques, such as snap fit, ratchet fit, interference fit, vacuum seal or other methods of attachment can be used to secure the base member 150 to the distal section 118 of the tubular body 110.

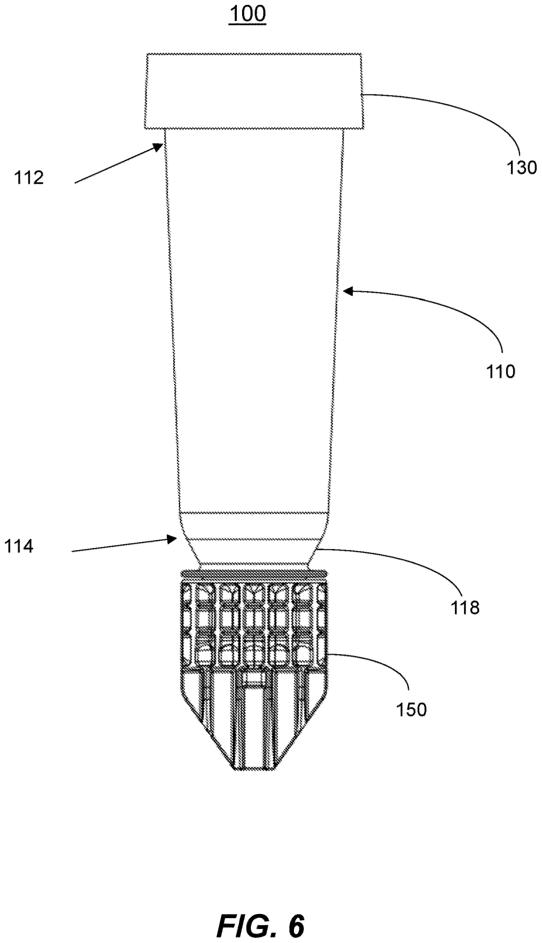

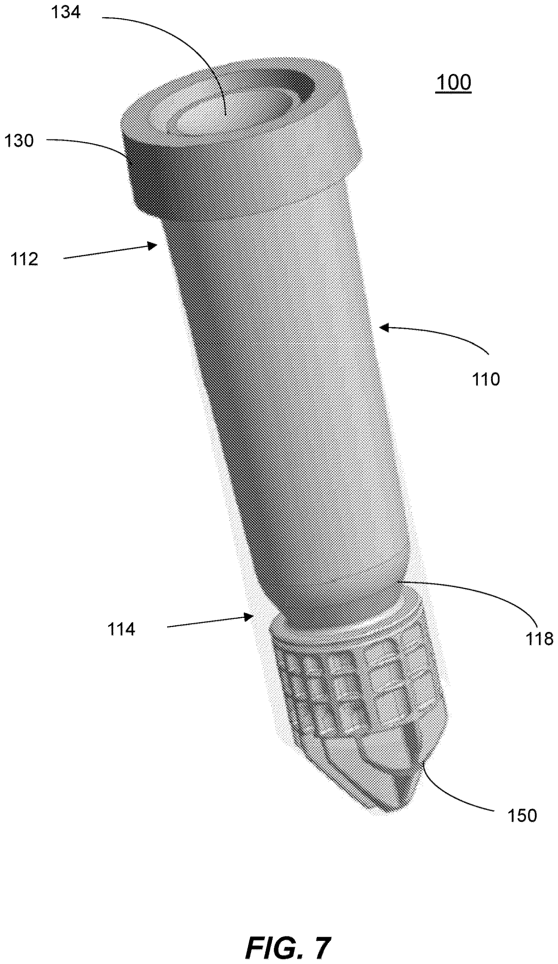

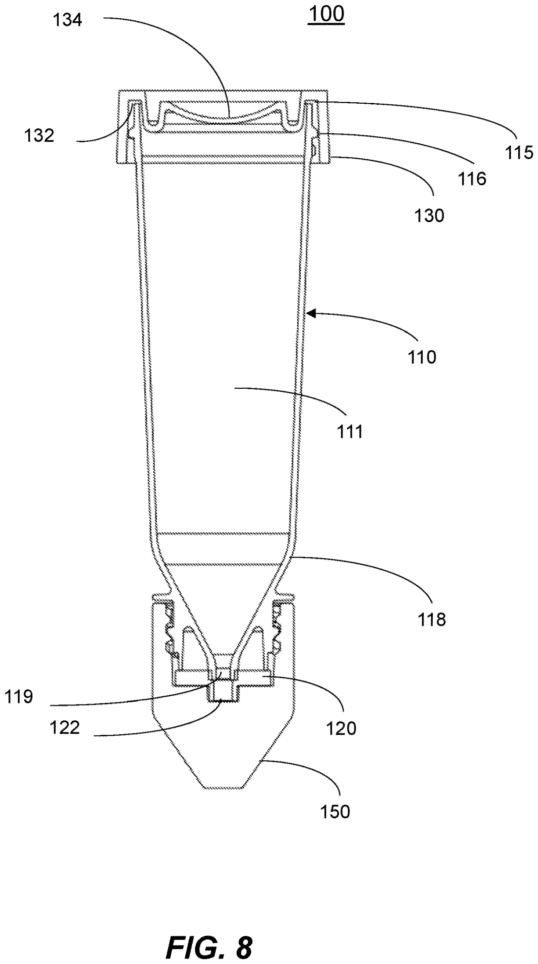

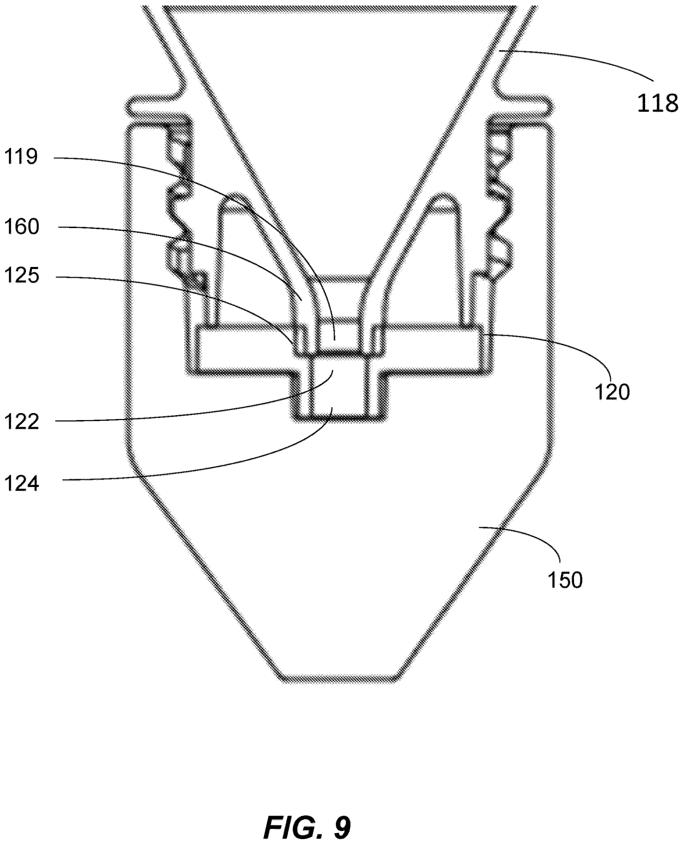

FIGS. 6-7 illustrate a side view and a perspective view of cell block system 100, respectively, and FIGS. 8-9 illustrate cross-sectional views of the system 100, in which the base member 150 is engaged to the distal section 118, to enclose the filter assembly 120 in the interior space 156 (see FIG. 5) between the base 150 and the distal section 118. The inner flange 160 at the distal section 118 cooperates with opening 125 in the filter assembly 120 to allow the biological sample to be received in the well 122. In this configuration, passage of liquid out of the filter assembly 120 through the filter membrane 124 is prevented by the solid nature of the surface 152 of the base member 150.

In the configurations shown in FIGS. 8-9, the filter assembly 120 is ready to receive a biological sample through distal outlet 119. After the sample has been introduced into the cell block apparatus 100, lid 130 can be secured to the tubular body 110 by engaging thread(s) 116. Alternatively, the sample can be delivered via a needle such as a hypodermic needle or an aspiration needle through a lid 130 configured with a puncturable, resealable center portion 134 that is already engaged with the tubular body 110. In either case, the biological sample is securely sealed within the cell block system 100 for storage and/or transport from the locus where it is taken, such as an examination room or an operating room, to the locus where it can be analyzed, such as a pathology lab. The sealed cell block apparatus can be subjected to centrifuging process(es) without any of the sample escaping the sample loading chamber and passing through the cell block filter assembly 120. Accordingly, the biological sample is separated or stratified (i.e. fluid content disposed above solid content) during the centrifuging process.

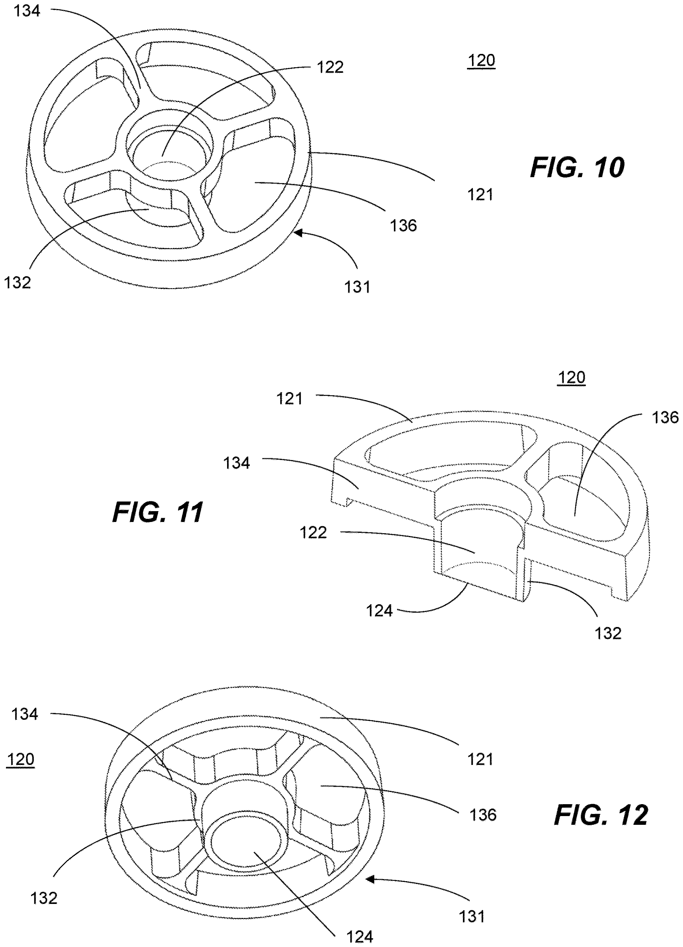

Referring to FIGS. 10-12, cell block filter assembly 120 is illustrated in greater detail. In an exemplary embodiment, the filter assembly 120 comprises a body portion 131 defining a well 122. The body portion 131 is typically a non-porous member. A filter membrane 124 is disposed within the well 122 of the body portion 131. In some embodiments, the body portion 131 includes a hub portion 132, a plurality of spokes 134 and a border 121. The configuration provides a plurality of openings 136 that allow the tissue sample formed within the filter assembly 122 to be secured in the wax or other medium during the slicing process discussed below.

In some embodiments, the body portion is made of linear low-density polyethylene (LLDPE) material. For example, the body portion can be fabricated by an injection mold process. In some embodiments, the body portion is fabricated substantially of LLDPE. In further embodiments, the body portion is fabricated from low-density polyethylene (LDPE), polyvinyl alcohol (PVA), or a blend of LLDPE, LDPE, and/or PVA or other similar materials.

In some embodiments, the filter membrane 124 is fabricated from a porous membrane, such as ePTFE or track etched polyethylene terephthalate (PET), for example. The membrane is specified so as to be small enough to prevent sample loss, but large enough for sufficient reagent infiltration into the sample. In some embodiments, the membrane 124 is attached to the base member 131 by heat sealing, such as thermal bonding. In some embodiments, the membrane is secured by ultrasonic welding, or by an adhesive.

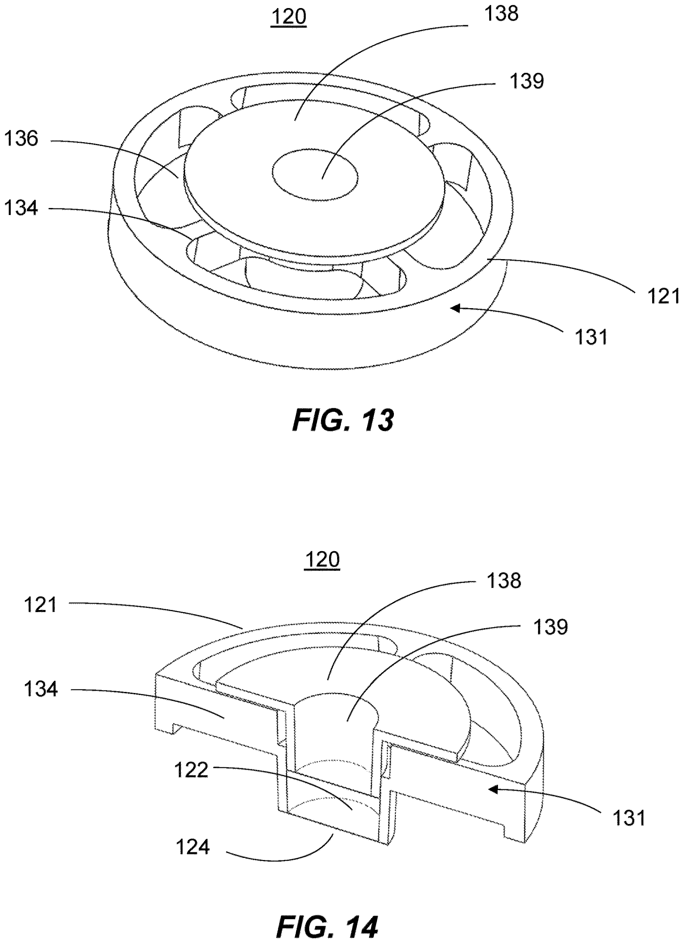

In some embodiments, the filter assembly 120 is further provided with a cover member 138 defining a central aperture 139 in which biological sample can flow into the well 122. (See FIGS. 13-14). In some embodiments, cover member 138 is fabricated from the same materials discussed above for the body portion 131, e.g., LLDPE. In some embodiments, aperture 139 supports a membrane, significantly as described as above for membrane 124. When installed in the aperture 139, the membrane filters the material before entering the well 122.

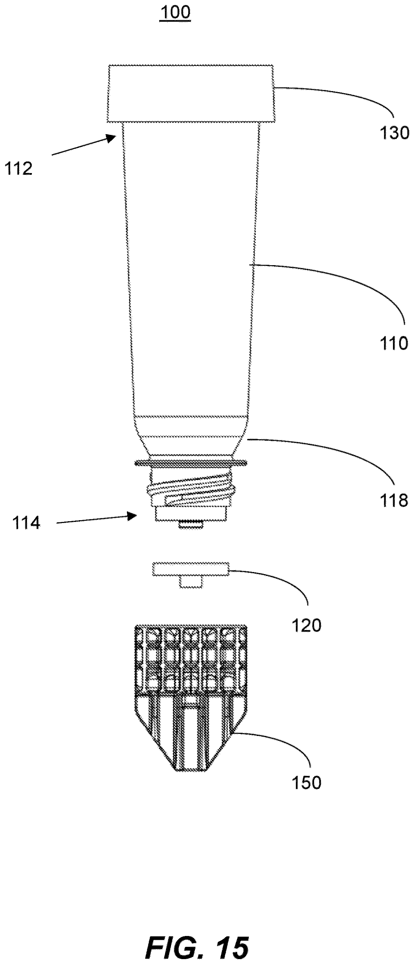

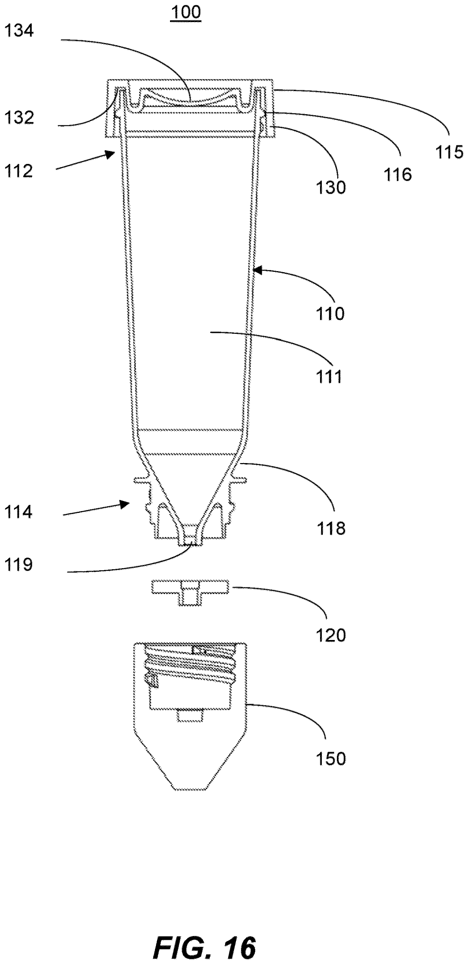

Following the tissue processing steps above, e.g., the operation of a centrifuge for stratification/separation of the cells in the biological sample from any liquid to isolate and consolidate the cells into a concentrated pellet by centrifugation, the base 150 and filter assembly are removed from the apparatus 100, as illustrated in FIGS. 15-16. As discussed above, the airtight seal, e.g., provided by the attachment of the lid 130 with the body 110, and by the sizing of the distal outlet 119, retain a substantial portion of the liquid within the loading compartment 111 of the body for subsequent reuse.

The sample, as well as the filter assembly 120, is then placed in mold and infiltrated with paraffin wax for subsequent sectioning on a microtome, for example. Thus, the entire filter assembly 120, including filter membrane 124 and the body portion 131 and 138, is sliceable by a microtome to form slices of cell block (and filter assembly) for mounting on a glass slide for analysis on a microscope or for other analyses such as microcellular analysis, e.g., DNA, RNA, and/or protein.

The materials of the body portion 131 and membrane 124 are particularly selected for their chemical compatibility and sectionability. Regarding chemical compatibility, no significant quantity of material dissolves when exposed to typical tissue processing reagents and temperatures. For example, the materials are exposed to ethanol, xylene and formalin, up to about 60°C. Regarding sectionability, the materials, e.g., LLDPE and ePTFE, are sliceable together with the contained cell block such that sufficiently thin slices can be achieved with a microtome for use in microscopy and other laboratory techniques. Moreover, the sectionability characteristics of the materials provide for insignificant wear on the microtome blade, and that subsequent slices are uniform, and mechanically stable, e.g., they do not break apart during slicing and subsequent handling.

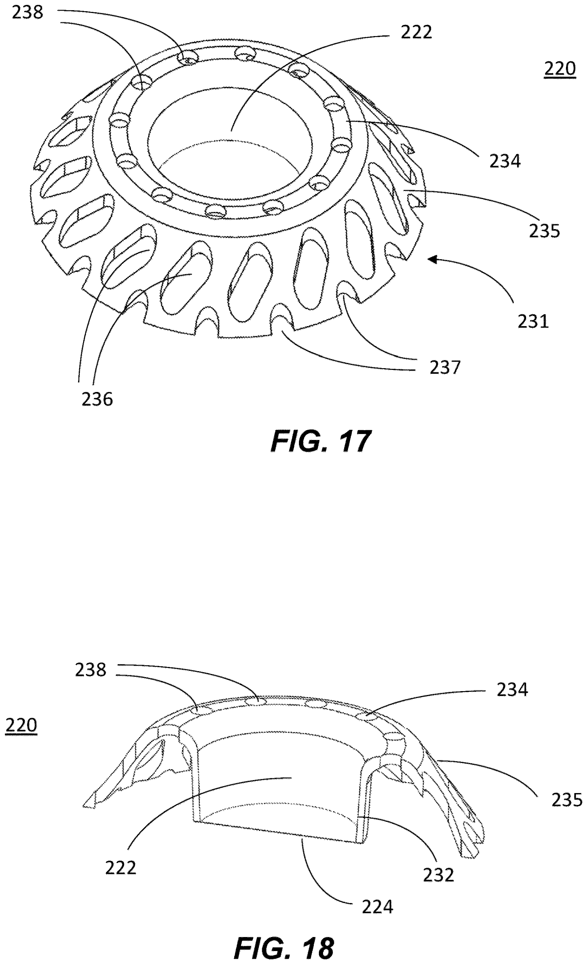

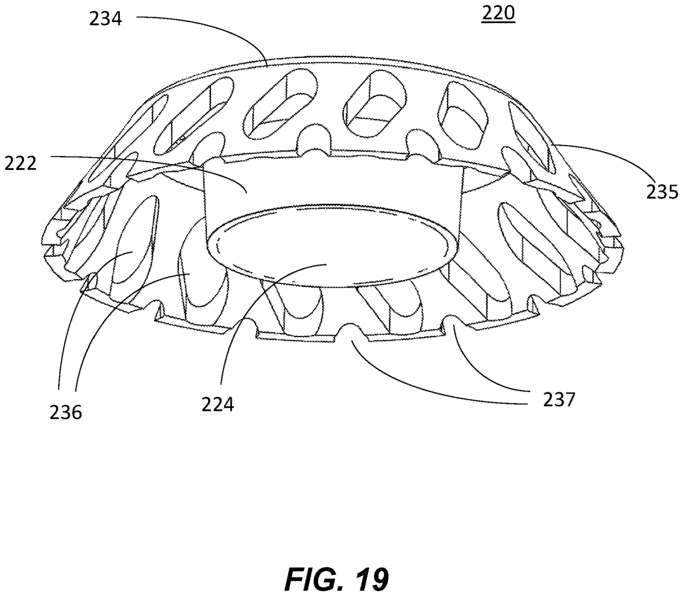

Referring to FIGS. 17-19, a further embodiment of cell block filter assembly 220 is illustrated. Cell block filter assembly 220 can be used in connection with cell block system 100 in the same manner as cell block filter assembly 120. The filter assembly 220 comprises a body portion 231 defining a well 222. The body portion 231 is typically a non-porous member, and is fabricated of the same materials as body portion 131, e.g., LLDPE, LDPE, PVA, or a combination thereof. A filter membrane 224 is disposed within the well 222 of the body portion 231, and is fabricated of the same materials as membrane 124, e.g., ePTFE. In some embodiments, the body portion 231 includes a hub portion 232 that defines the well 222. A top wall portion 234 extends peripherally from the hub portion 232 and defines a plurality of holes 238. A conical or tapered wall portion 235 extends from the top wall portion 234 and also defines a plurality of holes 236. The plurality of partially cut-outs or serrations 237 are formed about the periphery of tapered wall portion 235. The openings 236, 237 and 238 allow the filter assembly 220 (and thus the tissue sample formed within) to be secured in the wax or other medium during the slicing process.

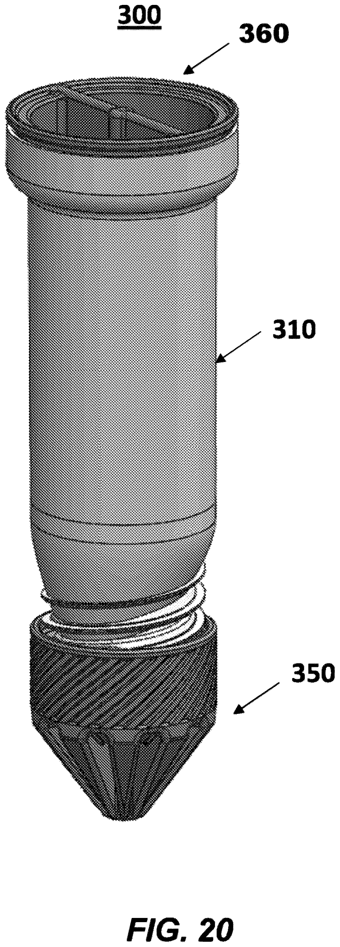

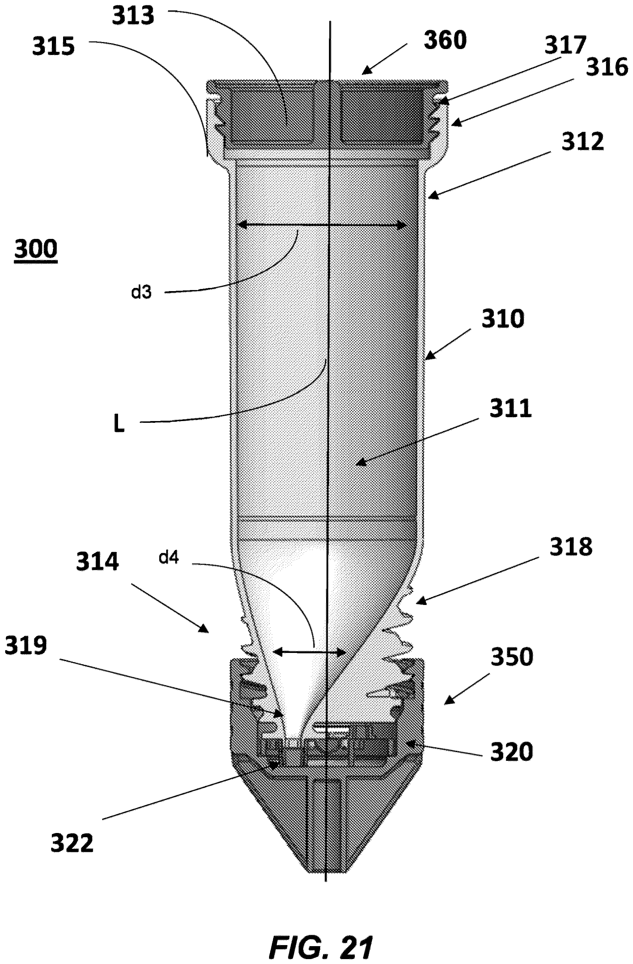

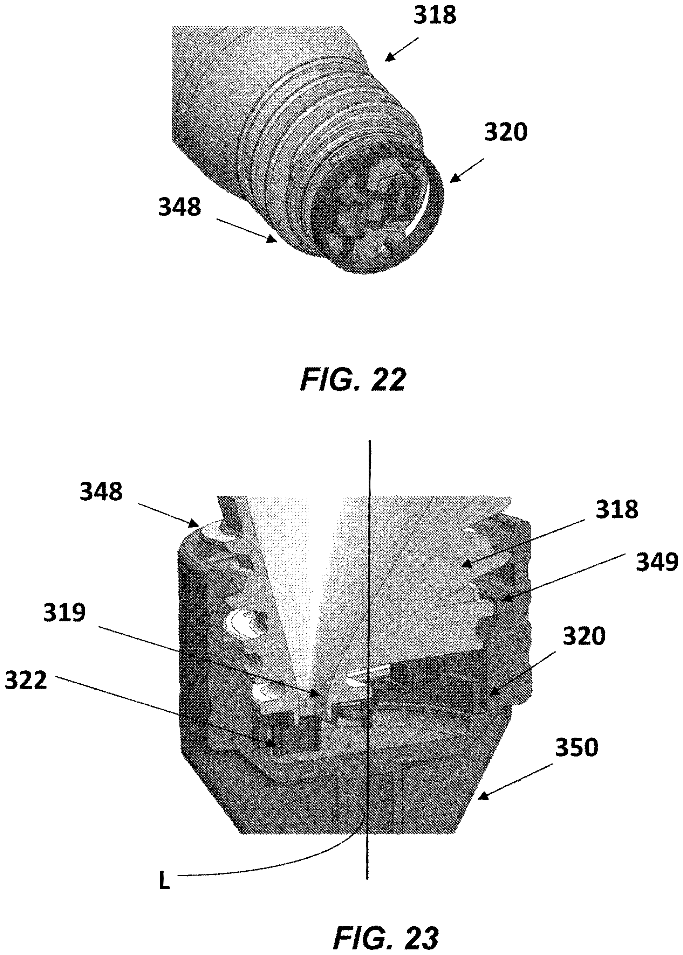

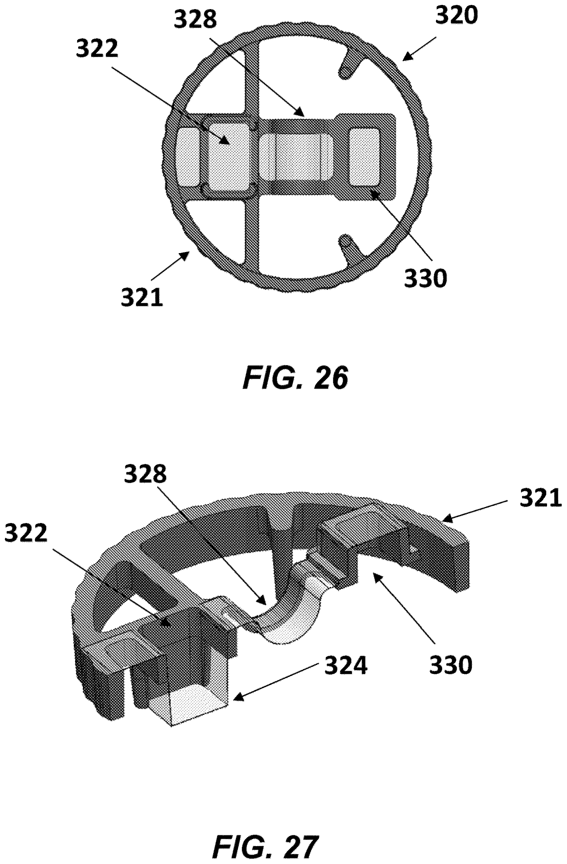

A further exemplary embodiment of the cell block system is shown schematically in FIGS. 20-27. Cell block system 300 includes an elongate tubular body 310, a cell block filter assembly 320, and a base member 350. The elongate tubular body 310 defines an interior loading chamber 311, and has a proximal end 312 and a distal end 314. In some embodiments, the elongate tubular body has a first diameter d3 at the proximal end 312 and a second diameter d4 at the distal end 314, wherein the second diameter d4 is smaller than the first diameter d3, as will be discussed in greater detail herein. A section 318 disposed between the proximal end 312 and the distal end 314 of the elongate tubular body 310, has a decreasing diameter along a length thereof to define a generally conical configuration, and further includes structure for receiving the cell block filter assembly as will be described below. As shown in FIGS. 21 and 23, the tubular body narrows in an offset fashion, such that the distal outlet 319 is offset from the longitudinal axis L of the elongated body 310.

With continued reference to FIG. 21, the elongate tubular body 310 defines an opening 313 at the proximal end 312 of the body (top of the figure). The opening 313 is closed by a cover 360, which provides a substantially airtight seal with the rim 315. The lid 360 can be configured with external screw thread or threads 317 to engage a thread or threads 316 disposed on the inside surface of a proximal section 312 of the elongate tubular body 310. However, other suitable methods and features can be used to engage the cover and elongate tubular body, such as a snap fit, ratchet fit, interference fit, vacuum seal or other methods of engagement, as would be appreciated by one of ordinary skill in the art.

In some embodiments, the elongate tubular body is preloaded with a fixative in the loading chamber. A "fixative" as used herein refers to a compound, such as formalin, ethanol, methanol, RPMI, saline for preservation and/or transportation of the sample.

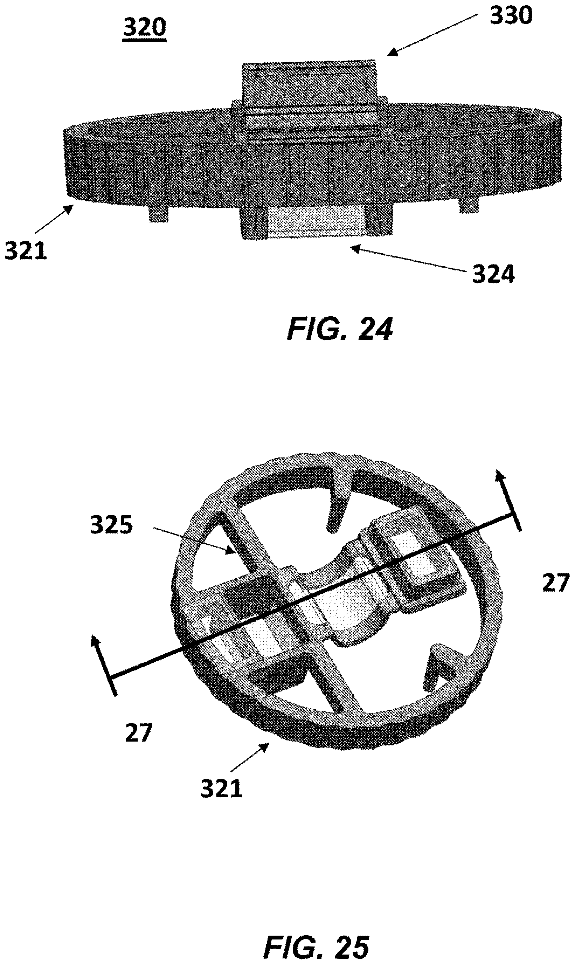

As illustrated in FIGS. 22-23, at the distal section 318 of the elongate tubular body 310 is configured to permit the engagement and secure mounting of the cell block filter assembly 320 thereto. As illustrated in FIGS. 24-27, the filter assembly 320 defines a border 321 circumscribing an open off-center well 322 in which a filter membrane 324 is disposed. The well 322 is configured with an opening sized to cooperate with the distal outlet 319 on the distal section 318 of the elongate tubular body 310. (See FIG. 23). In an embodiment, cells may be collected in the center well 322, such as after filtration, to provide a substantially cylindrical cell block.

The base member 350 is configured to support the cell block filter assembly 320, and to be attached to the distal section 318 of tubular body 310 to secure the assembly 320 thereto. In some embodiments, the outer surface of the distal section 318 can be configured with a thread or a plurality of threads 348 to engage with a thread or threads 349 on interior portion of the base member 350 to secure the base member 350 to the distal section 318 of the elongate tubular body 310, thereby stabilizing the cell block filter assembly 320 therein during centrifuging or other similar processes. It is contemplated that other techniques, such as snap fit, ratchet fit, interference fit, vacuum seal or other methods of attachment can be used to secure the base member 350 to the distal section 318 of the tubular body 310. In this configuration, passage of liquid out of the filter assembly 320 through the filter membrane 324 is prevented by the solid nature of the surface of the base member 350.

In the configurations shown in FIGS. 24-27, the filter assembly 320 is ready to receive a biological sample through distal opening of elongate body 310. After the sample has been introduced into the cell block apparatus 300, cap 360 can be secured to the tubular body 310. Alternatively, the sample can be delivered via a needle such as a hypodermic needle or an aspiration needle through a cap 360 configured with a puncturable, resealable center portion that is already engaged with the tubular body 310. In either case, the biological sample is securely sealed within the cell block system 300 for storage and/or transport from the locus where it is taken, such as an examination room or an operating room, to the locus where it can be analyzed, such as a pathology lab. The sealed cell block apparatus can be subjected to centrifuging process(es) without any of the sample escaping the sample loading chamber and passing through the cell block filter assembly 320. Accordingly, the biological sample is separated or stratified (i.e. fluid content disposed above solid content) during the centrifuging process.

Referring to FIGS. 24-27, cell block filter assembly 320 is illustrated in greater detail. In an exemplary embodiment, the filter assembly 320 includes a body portion 321 typically fabricated from a non-porous material and defines a well 322 that is off-center. A filter membrane 324 is disposed within the well 322. In some embodiments, support member(s) 325 extend from a border 321 to support the well 322. The filter assembly 320 further includes a flexible hinge 328 (also referred to as a "living hinge") and a cover portion 330. The hinge portion 328 can be fabricated from the same material as the body of the filter assembly, linear low-density polyethylene (LLDPE) material. The lid 330 is sized and configured to fit over the well 322. In some embodiments, the cover includes an aperture that is covered with the filter membrane material. In some embodiments, the filter membrane 324 is fabricated from a porous membrane, such as ePTFE or track etched polyethylene terephthalate (PET), for example. The membrane is specified so as to be small enough to prevent sample loss, but large enough for sufficient reagent infiltration into the sample. In some embodiments, the membrane 324 is attached by heat sealing, such as thermal bonding, to the lid 330 and the well 322 and covers an opening at the bottom portion of the well 322. In some embodiments, the membrane is secured by ultrasonic welding, or by an adhesive.

It is understood that the subject matter described herein is not limited to particular embodiments described, as such may, of course, vary. For example, the exemplary embodiments describe above are not limited to fine needle aspiration applications. Instead the disclosed subject matter is applicable to additional clinical settings such as processing small surgical biopsies (less than 2 cm), in research laboratories for isolating cells from bone marrow diluted by blood, analyzing small samples of engineered tissues, and purifying cells in a spin column. Accordingly, nothing contained in the Abstract or the Summary should be understood as limiting the scope of the disclosure. It is also understood that the terminology used herein is for the purpose of describing particular embodiments only, and is not intended to be limiting. Where a range of values is provided, it is understood that each intervening value between the upper and lower limit of that range and any other stated or intervening value in that stated range, is encompassed within the disclosed subject matter.

## Claims

1. A medical apparatus comprising:
an elongated body (310) having a proximal end (312), a distal end (314), and defining a loading chamber (311) between the proximal end and the distal end for storing a liquid therein, the elongated body defining an outlet (319) at the distal end thereof;
a cell block filter assembly (320) comprising:
a cell block body (321) defining a well portion (322) matingly engageable with the distal end of the elongated body for receiving fluid from the outlet of the elongated body and defining a well opening on a bottom thereof;
a filter membrane (324) disposed across the well opening within the well portion; and
a cover member (330) positionable over the well portion; and
a base member (350) for securing the cell block filter assembly (320) at the distal end (314) of the elongated body (310), and wherein the outer surface of the distal end of the elongated body is configured to engage with the base member;
wherein the entire cell block filter assembly is sliceable into slices having a thickness suitable for mounting on a laboratory slide; and
wherein the base member (350) prevents the passage of fluid out of the filter assembly (320).

2. The medical apparatus of claim 1, wherein the base member (350) is secured to the elongated body (310) with threaded engagement, friction fit, or a ratchet engagement.

3. The medical apparatus of claim 1, wherein the cell block body (321) is fabricated substantially from LLDPE.

4. The medical apparatus of claim 1, wherein the cover member (330) is fabricated substantially from LLDPE.

5. The medical apparatus of claim 1, wherein the cover member (330) defines a cover opening, the cover member further comprising a filter membrane disposed across the cover opening.

6. The medical apparatus of claim 5, wherein the cover member (330) comprises a hinge portion (328) for connecting the cover member to the cell block body.

7. The medical apparatus of claim 6, wherein the hinge portion (328) is a living hinge.

8. The medical apparatus of claim 1, wherein the filter membrane (324) is fabricated substantially from ePTFE or PET.

9. The medical apparatus of claim 1, wherein the distal end (314) of the elongated body (310) defines a smaller diameter than the proximal end (312) to define a generally conical portion.

10. The medical apparatus of claim 1, wherein the outlet (319) is offset from the longitudinal axis of the elongated body (310).

11. The medical apparatus if claim 1, further comprising a cap (360) disposed on the proximal end (312) of the elongated body (310), the cap configured to engage the proximal end of the elongated body to form an airtight seal between the cap and the elongated body.

12. The medical apparatus of claim 11, where the cap (360) is threadedly engaged with the elongated body (310) with threads formed on the outer surface of the cap and threads formed on the inner surface of the proximal end (312) of the elongated body.

13. The medical apparatus of claim 11, wherein the cap (330) is provided with a puncturable, resealable portion.

## Patentansprüche

1. Medizinische Vorrichtung, umfassend:
einen länglichen Körper (310) mit einem proximalen Ende (312) und einem distalen Ende (314), der zwischen dem proximalen Ende und dem distalen Ende eine Ladekammer (311) zum Speichern einer Flüssigkeit darin definiert, wobei der längliche Körper an seinem distalen Ende einen Auslass (319) definiert;
eine Zellblockfilteranordnung (320), umfassend:
einen Zellblockkörper (321), der einen Schachtabschnitt (322) definiert, der mit dem distalen Ende des länglichen Körpers in Eingriff gebracht werden kann, um Flüssigkeit aus dem Auslass des länglichen Körpers aufzunehmen, und der an seiner Unterseite eine Schachtöffnung definiert;
eine Filtermembran (324), die über der Schachtöffnung innerhalb des Schachtabschnitts angeordnet ist; und
ein Abdeckelement (330), das über dem Schachtabschnitt positionierbar ist; und
ein Basiselement (350) zum Sichern der Zellblockfilteranordnung (320) am distalen Ende (314) des länglichen Körpers (310), und wobei die Außenfläche des distalen Endes des länglichen Körpers so konfiguriert ist, dass sie mit dem Basiselement in Eingriff steht;
wobei die gesamte Zellblockfilteranordnung in Scheiben mit einer Dicke schneidbar ist, die für die Montage auf einem Laborobjektträger geeignet ist; und
wobei das Basiselement (350) den Austritt von Flüssigkeit aus der Filteranordnung (320) verhindert.

2. Medizinische Vorrichtung nach Anspruch 1, wobei das Basiselement (350) mit Gewinde, Reibungspassung oder Ratscheneingriff am länglichen Körper (310) befestigt ist.

3. Medizinische Vorrichtung nach Anspruch 1, wobei der Zellblockkörper (321) im Wesentlichen aus LLDPE hergestellt ist.

4. Medizinische Vorrichtung nach Anspruch 1, wobei das Abdeckelement (330) im Wesentlichen aus LLDPE hergestellt ist.

5. Medizinische Vorrichtung nach Anspruch 1, wobei das Abdeckelement (330) eine Abdecköffnung definiert, wobei das Abdeckelement ferner eine Filtermembran umfasst, die über der Abdecköffnung angeordnet ist.

6. Medizinische Vorrichtung nach Anspruch 5, wobei das Abdeckelement (330) einen Scharnierabschnitt (328) zum Verbinden des Abdeckelements mit dem Zellblockkörper umfasst.

7. Medizinische Vorrichtung nach Anspruch 6, wobei der Scharnierabschnitt (328) ein bewegliches Scharnier ist.

8. Medizinische Vorrichtung nach Anspruch 1, wobei die Filtermembran (324) im Wesentlichen aus ePTFE oder PET hergestellt ist.

9. Medizinische Vorrichtung nach Anspruch 1, wobei das distale Ende (314) des länglichen Körpers (310) einen kleineren Durchmesser als das proximale Ende (312) aufweist, um einen im Allgemeinen konischen Abschnitt zu definieren.

10. Medizinische Vorrichtung nach Anspruch 1, wobei der Auslass (319) von der Längsachse des länglichen Körpers (310) versetzt ist.

11. Medizinische Vorrichtung nach Anspruch 1, ferner umfassend eine Kappe (360), die am proximalen Ende (312) des länglichen Körpers (310) angeordnet ist, wobei die Kappe so konfiguriert ist, dass sie mit dem proximalen Ende des länglichen Körpers in Eingriff kommt, um eine luftdichte Abdichtung zwischen der Kappe und dem länglichen Körper zu bilden.

12. Medizinische Vorrichtung nach Anspruch 11, wobei die Kappe (360) mit dem länglichen Körper (310) verschraubt ist, wobei an der Außenfläche der Kappe Gewinde ausgebildet sind und an der Innenfläche des proximalen Endes (312) des länglichen Körpers Gewinde ausgebildet sind.

13. Medizinische Vorrichtung nach Anspruch 11, wobei die Kappe (330) mit einem durchstechbaren, wiederverschließbaren Abschnitt versehen ist.

## Revendications

1. Appareil médical comprenant :
un corps allongé (310) présentant une extrémité proximale (312), une extrémité distale (314), et définissant une chambre de chargement (311) entre l'extrémité proximale et l'extrémité distale pour y stocker un liquide, le corps allongé définissant une sortie (319) au niveau de l'extrémité distale de celui-ci ;
un ensemble de filtre de bloc cellulaire (320) comprenant :
un corps de bloc cellulaire (321) définissant une partie de puits (322) pouvant venir en prise de manière complémentaire avec l'extrémité distale du corps allongé pour recevoir du fluide provenant de la sortie du corps allongé et définissant une ouverture de puits sur un fond de celui-ci ;
une membrane de filtre (324) disposée à travers l'ouverture du puits à l'intérieur de la partie de puits ; et
un élément de couvercle (330) pouvant être positionné sur la partie de puits ; et
un élément de base (350) pour fixer l'ensemble de filtre de bloc cellulaire (320) au niveau de l'extrémité distale (314) du corps allongé (310), et dans lequel la surface extérieure de l'extrémité distale du corps allongé est conçue pour venir en prise avec l'élément de base ;
dans lequel l'ensemble de filtre de bloc cellulaire peut être découpé en tranches présentant une épaisseur adaptée au montage sur une lame de laboratoire ; et
dans lequel l'élément de base (350) empêche le passage du fluide hors de l'ensemble de filtre (320).

2. Appareil médical selon la revendication 1, dans lequel l'élément de base (350) est fixé au corps allongé (310) avec une prise filetée, un ajustement par friction ou une prise à cliquet.

3. Appareil médical selon la revendication 1, dans lequel le corps de bloc cellulaire (321) est fabriqué essentiellement à partir de LLDPE.

4. Appareil médical selon la revendication 1, dans lequel l'élément de couvercle (330) est fabriqué essentiellement à partir de LLDPE.

5. Appareil médical selon la revendication 1, dans lequel l'élément de couvercle (330) définit une ouverture de couvercle, l'élément de couvercle comprenant en outre une membrane de filtre disposée à travers l'ouverture de couvercle.

6. Appareil médical selon la revendication 5, dans lequel l'élément de couvercle (330) comprend une partie de charnière (328) pour relier l'élément de couvercle au corps de bloc cellulaire.

7. Appareil médical selon la revendication 6, dans lequel la partie de charnière (328) est une charnière mobile.

8. Appareil médical selon la revendication 1, dans lequel la membrane de filtre (324) est fabriquée essentiellement à partir d'ePTFE ou de PET.

9. Appareil médical selon la revendication 1, dans lequel l'extrémité distale (314) du corps allongé (310) définit un diamètre plus petit que l'extrémité proximale (312) pour définir une partie généralement conique.

10. Appareil médical selon la revendication 1, dans lequel la sortie (319) est décalée par rapport à l'axe longitudinal du corps allongé (310).

11. Appareil médical selon la revendication 1, comprenant également un capuchon (360) disposé sur l'extrémité proximale (312) du corps allongé (310), le capuchon étant conçu pour venir en prise avec l'extrémité proximale du corps allongé pour former une fermeture hermétique entre le capuchon et le corps allongé.

12. Appareil médical selon la revendication 11, dans lequel le capuchon (360) est en prise fileté avec le corps allongé (310) avec des filetages formés sur la surface extérieure du capuchon et des filetages formés sur la surface intérieure de l'extrémité proximale (312) du corps allongé.

13. Appareil médical selon la revendication 11, dans lequel le capuchon (330) est pourvu d'une partie pouvant être perforée et refermée.
